(19) Europäisches Patentamt
European Patent Office
Office européen des brevets

(11) EP 2 666 459 A1

(12) **EUROPEAN PATENT APPLICATION**
published in accordance with Art. 153(4) EPC

(43) Date of publication:
**27.11.2013 Bulletin 2013/48**

(21) Application number: **12736973.4**

(22) Date of filing: **18.01.2012**

(51) Int Cl.:
***A61K 8/36*** *(2006.01)* ***A61K 8/14*** *(2006.01)*
***A61K 8/34*** *(2006.01)* ***A61K 8/365*** *(2006.01)*
***A61K 8/41*** *(2006.01)* ***A61Q 5/12*** *(2006.01)*

(86) International application number:
**PCT/JP2012/000283**

(87) International publication number:
**WO 2012/098877 (26.07.2012 Gazette 2012/30)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.01.2011 JP 2011009269**

(71) Applicant: **Kao Corporation**
**Chuo-Ku**
**Tokyo 103-8210 (JP)**

(72) Inventors:
- **TETSU, Makio**
  **Tokyo 131-8501 (JP)**
- **SUZUKI, Ryosuke**
  **Tokyo 131-8501 (JP)**

(74) Representative: **HOFFMANN EITLE**
**Patent- und Rechtsanwälte**
**Arabellastrasse 4**
**81925 München (DE)**

# (54) PROCESS FOR PRODUCING VESICLE COMPOSITION

(57) The present invention relates to a process for preparing a vesicle composition having a continuous phase constituted of an aqueous phase, including: melting an oil phase at a temperature of equal to or higher than a melting point of the oil phase, the oil phase containing following components (A) to (D): component (A) straight chain fatty acid having 14 to 24 carbon atoms; component (B) branched fatty acid having 14 to 24 carbon atoms and having a certain structure; component (C) tertiary amine compound; and component (D) organic acid having 1 to 8 carbon atom(s), mass ratio of the component (A) and the component (B) being: (A)/(B) = 70/30 to 40/60, and adding the melted oil phase to the aqueous phase while mixing thereof.

EP 2 666 459 A1

## Description

TECHNICAL FIELD

The present invention relates to a process for preparing a vesicle composition.

BACKGROUND ART

In recent years, utilizations of a permanent, a hair color, a hair bleach and the like are generalized, and on the other hand, hair damages associated with such chemical treatments are problematic. Hair cosmetics such as a rinse, a conditioner, a treatment and the like are conventionally used for improving a feel of the hair after shampooing, and further improvement in performances is desired in view of reducing the damage of the hair.

Patent Document 1 discloses a hair cosmetic, which is capable of repairing or suppressing a hair damage and fatigue breaking due to chemical treatments, drying with a dryer and daily hair-care activities, and also capable of providing improved softness and supple feel from the wetted condition through the dried condition. Patent Document 1 also discloses a specific embodiment for solving such problem, which is directed to a hair cosmetic containing a specific branched fatty acid or its salt and a specific tertiary amine or its salt, and additionally a specific aromatic alcohol as required.

Patent Document 2 discloses a hair cosmetic having biodegradability and being environmentally-friendly, and being capable of stabilizing a composition containing blended long-chain alcohol, oil agent and the like. It is described that such hair cosmetic provides enhanced flexibility and smoothness in the wetted condition. Patent Document 2 also disclose a specific embodiment for solving such problem, which is a hair cosmetic containing a specific tertiary amine, a linear or branched fatty acid, an inorganic acid or an organic acid.

Also, Patent Document 3 discloses a hair treatment composition constituted of a multilamellar vesicle dispersion containing cholesterol, a basic amino acid and a fatty acid, and stabilized by a nonionic surfactant, and a hair treatment composition such as a shampoo or a conditioner containing the multilamellar vesicle dispersion, for the purpose of repairing and preventing hair damage. It is stated that this treatment composition specifically promotes penetration of some hair-treatment effective materials into hair fibers.

As one method for producing an emulsion composition, a phase inversion emulsification method or a liquid crystal emulsification method is known. The phase inversion emulsification method is a method in which emulsification is carried out while adding an aqueous phase to an oil phase. The liquid crystal emulsification method is a method in which emulsification is carried out while adding an aqueous phase to a liquid crystal phase. For example, in Non-Patent Document 1, a fine emulsion having an average particle size of equal to or smaller than 1 μm is formed by emulsifying while adding an aqueous phase in an oil phase and by utilizing a great reduction in the oil/water interfacial tension around the phase inversion point.

Also, for example, Patent Document 4 discloses a process for preparing an oil in an aqueous emulsion containing quaternary ammonium salt, which exhibits enhanced long-lasting and diffusibility of an aroma, and also achieves an improved temporal stability.

RELATED DOCUMENTS

PATENT DOCUMENTS

[Patent Document 1]
Japanese Laid-Open Patent Application Publication No. 2008-297,218
[Patent Document 2]
Japanese Laid-Open Patent Application Publication No. 2002-114,648
[Patent Document 3]
Japanese Patent Domestic Publication No. 2002-516,831
[Patent Document 4]
Japanese Laid-Open Patent Application Publication No. 2008-094,980

NON-PATENT DOCUMENTS

[Non-Patent Document 1]

Optimization of Nano-emulsion Preparation by Low-Energy Methods in an Ionic Surfactant System, Langmuir 2006, 22, 8326-8332.

SUMMARY OF THE INVENTION

While Patent Documents 1 and 2 describe the hair cosmetics containing a combination of a specific tertiary amine and a linear or branched fatty acid, no reference describes a creation of a vesicle structure, and the technologies described in Patent Documents 1 and 2 cannot provide a sufficient effect in view of achieving inhibition to the slimy feel in the rinsing and inhibition to the pastiness after the drying.

On the other hand, the technology described in Patent Document 3 allows creating a multilamellar vesicle by coexisting cholesterol as an essential constituent. The vesicle structure is formed due to the presence of specific lipids such as sterols and the like co-existing therein. However, the technology described in Patent Document 3 cannot provide a sufficient effect in view of achieving the smoothness and inhibiting the slimy feel in the rinsing and also achieving the smoothness and inhibiting the pastiness after the drying, even if the components for an ordinary treatment agent are employed.

Further, while Non-Patent Document 1 and Patent Document 4 involve the phase inversion emulsification for stabilizing the emulsion, there is no reference related to the creation of the vesicle structure. Also, there is a problem in the emulsion concerning the slimy feel in the rinsing and the pastiness after the drying, and thus there are needs to be improved in the technologies described in Non-Patent Document 1 and Patent Document 4.

The present inventors have found that a vesicle having relatively larger mean particle diameter can be constituted in water by, in addition to employing a specific organic acid in combination with a specific tertiary amine and a specific branched fatty acid, and additionally a straight chain fatty acid.

Moreover, the present inventors have found that the hair cosmetic containing the composition, in which a vesicle occupies larger capacity in this way, achieves smoothness and prevents slimy feeling in the rinsing and achieves smoothness and inhibits the pastiness after the drying.

Such vesicle composition can, more specifically, be composed of components (A), (B), (C) and (D) and water, and can be produced with a specific tertiary amine and a specific branched fatty acid, and a straight chain fatty acid and a specific organic acid, and can also exhibit sufficiently larger volume fraction of the vesicle.

According to one aspect of the present invention, there is provided a process for preparing a vesicle composition having a continuous phase constituted of an aqueous phase, including:

melting an oil phase at a temperature of equal to or higher than the melting point of the oil phase, the oil phase containing the following components (A) to (D):

component (A) straight chain fatty acid having 14 to 24 carbon atoms;
component (B) branched fatty acid having 14 to 24 carbon atoms and represented by a general formula (1);
component (C) tertiary amine compound; and
component (D) organic acid having 1 to 8 carbon atom(s),
mass ratio of the component (A) and the component (B) being: (A)/(B) = 70/30 to 40/60, and

adding the melted oil phase to the aqueous phase while mixing thereof.

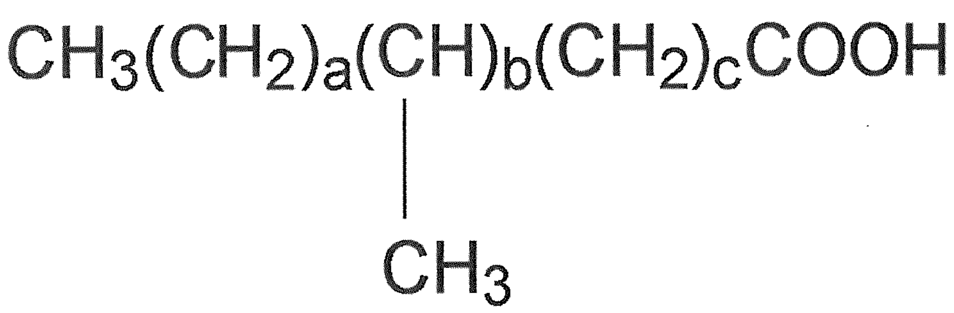

(1)

In the formula (1), summation of a, b and c is: a + b + c = 11 to 21, and b is 1.

According to another aspect of the present invention, there is provided a process for preparing a hair cosmetic, including:

melting an oil phase at a temperature of equal to or higher than a melting point of the oil phase, the oil phase containing the above-described components (A) to (D), mass ratio of the component (A) and the component (B) being: (A)/(B) = 70/30 to 40/60;
adding the melted oil phase to the aqueous phase while mixing thereof to produce a vesicle composition having the aqueous phase as a continuous phase; and
mixing the vesicle composition in a base mixture containing at least a cationic surfactant and an aliphatic alcohol.

According to further aspect of the present invention, there is provided a vesicle composition having a continuous phase constituted of an aqueous phase, the composition containing the above-described components (A), (B), (C) and (D) and water, wherein mass ratio of the component (A) and the component (B) is: (A)/(B) = 70/30 to 40/60, and mean particle diameter of the vesicle in the vesicle composition is 5 to 500 μm.
According to yet another aspect of the present invention, there is provided a hair cosmetic in a form of a treatment, a conditioner or a rinse, which is obtainable by mixing the above-described vesicle composition to a base mixture containing one or more types of cationic surfactant(s) and an aliphatic alcohol.
According to the present invention, the slimy feel can be reduced while providing the smoothness for the hair in the rinsing, and the pastiness can be reduced while providing the smoothness for the hair after the drying.

DESCRIPTION OF THE INVENTION

The vesicle composition of the present invention can be obtained through the following steps.
More specifically, the vesicle composition of the present invention can be preferably produced by steps of

(I) dissolving an oil phase containing the component (A), the component (B), the component (C), and the component (D) at a temperature of equal to or higher than a melting point of the oil phase; and
(II) adding an aqueous phase to the dissolved oil phase while mixing thereof. The vesicle composition having the continuous phase, which is the aqueous phase, can be obtained according to such procedure. It is preferable that such vesicle composition is constituted in the form of a dispersion solution (premix) of the vesicle.

The component (A) used in the present invention is a straight chain fatty acid having 14 to 24 carbon atoms. The component (A) may be either a saturated fatty acid or an unsaturated fatty acid, and may more preferably be a saturated fatty acid. In view of creating the vesicle, the carbon number of the component (A) is equal to or larger than 14 and more preferably equal to or larger than 16, and equal to or smaller than 24 and more preferably equal to or smaller than 22 and further preferably equal to or smaller than 20. Among these, in view of enhancing the smoothness in the rinsing, stearic acid which has 18 carbon atoms is preferable.
The content of the component (A) in the vesicle composition is preferably equal to or higher than 0.5 % by mass and more preferably equal to or higher than 1 % by mass, and preferably equal to or lower than 5 % by mass and more preferably equal to or lower than 3 % by mass.
The component (B) employed in the present invention is a branched fatty acid having 14 to 24 carbon atoms, and also a branched fatty acid represented by the above-described general formula (1).
Typical branched fatty acid includes 14-methyl pentadecanoic acid, 15-methyl hexadecanoic acid, 16-methyl heptadecanoic acid, 17-methyl octadecanoic acid, 18-methyl nonadecanoic acid, 19-methyl eicosanoic acid, 20-methyl heneicosanoic acid, 14-methyl hexadecanoic acid, 15-methyl heptadecanoic acid, 16-methyl octadecanoic acid, 17-methyl nonadecanoic acid, 18-methyl eicosanoic acid, and 19-methyl heneicosanoic acid. In addition to above, "Cosmetics Raw Material Standard", the Second Edition, Explanatory Note I (1984), YAKUJI NIPPO LIMITED, p. 87(C) describes that isostearic acid is C18 branched-chain fatty acid having a methyl group as a side chain and obtained by conducting hydrogenation to an unsaturated side chain fatty acid created as a byproduct in the synthesis of a dimer acid from oleic acid, though the hydrogenating position is not specified, and typical commercially available product thereof may be a mixture of C18 branched-chain fatty acids of different substitutional positions of, for example, ISOSTEARIC ACID EX [commercially available from KOKYU ALCOHOL KOGYO CO., LTD.].
In the above-described formula (1), the summation of a+b+c is, in view of providing enhanced smoothness in the rinsing, preferably equal to or higher than 13, and is also preferably equal to or lower than 19 and more preferably equal to or lower than 17. In other words, the carbon number of the component (B) is preferably equal to or higher than 16, and is also preferably equal to or lower than 22 and more preferably equal to or lower than 20. Among these, in view of achieving the feel of use without slimy touch in the rinsing, isostearic acid having one methyl branch and in which the summation: a+b+c is 15 in the above-described formula (1), is preferable for the component (B).
Two or more types of branched fatty acids may be jointly employed for the component (B). The content of the component (B) in the vesicle composition is preferably equal to or higher than 0.2 % by mass and more preferably equal to or higher than 0.5 % by mass, and is preferably equal to or lower than 4 % by mass and more preferably equal to or

lower than 2 % by mass.

The mass ratio of the component (A) and the component (B) ((A) / (B)) is 70/30 to 40/60, and in view of providing larger vesicle particle diameter of equal to or larger than 5 μm, achieving the smoothness and reducing the slimy feel in the rinsing, and providing the smoothness and reducing the pastiness after the drying, it is preferable to employ 60/40 to 50/50.

The component (A) and the component (B) may be preferably combined at a formulation, at which the congealing point of the mixture of the component (A) and the component (B) is equal to or higher than 40 degrees C, in view of creating the vesicle, and may also be preferably combined at a formulation for achieving the congealing point of equal to or lower than 55 degrees C, in view of providing the smoothness and avoiding the slimy feel in the rinsing, and providing the smoothness and reducing the pastiness after the drying. More preferably, the component (A) may be combined with the component (B) at the formulation, which provides the congealing point of equal to or higher than 45 degrees C and equal to or lower than 52 degrees C. In addition to above, the congealing point can be measured by the method described in Examples as discussed later.

A combination of the compounds of the same carbon number may be preferably employed as the combination of the component (A) and the component (B), in view of achieving the smoothness and reducing the slimy feel in the rinsing, and in view of providing the smoothness and reducing the pastiness after the drying. More specifically, a combination of linear stearic acid with 16-methyl heptadecanoic acid, or a combination of linear stearic acid and isostearic acid (specifically, ISOSTEARIC ACID EX) is preferable.

Typical example of the component (C) employed in the present invention includes tertiary amine compounds represented by the general formula (2).

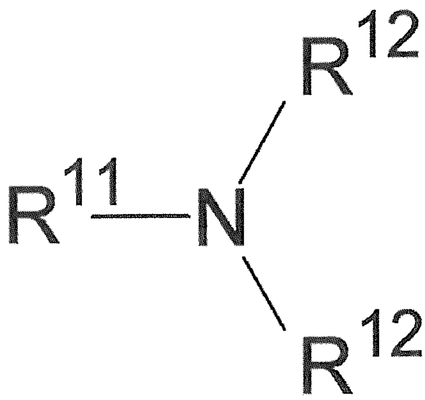

(2)

In the above-described general formula (2), $R^{11}$ represents a linear or branched alkyl group, alkenyl group, or aliphatic acyloxy(polyethoxy)ethyl group having 8 to 35 carbon atoms, which may be divided by a functional group represented by -OCO- or -COO- or may be substituted with -OH. In the above-described general formula (2), $R^{12}$ represents alkyl group or hydroxyalkyl group having 1 to 22 carbon atom(s), or polyoxyethylene group of total addition mol number of equal to or smaller than 10, and two of $R^{12}$ may be the same or different.

It is preferable for the tertiary amine compound of the component (C) to contain one, two or more of the tertiary amine compounds of the following (i) to (iii).

(i) hydroxyether alkyl amine

For example, compounds represented by the following general formula (3) are included.

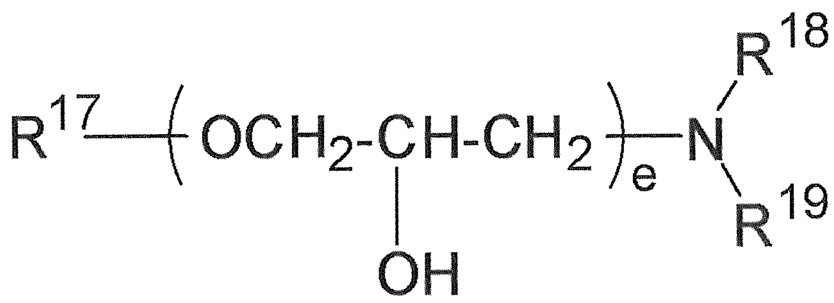

(3)

In the above-described general formula (3), $R^{17}$ represents a linear or branched alkyl group or alkenyl group having 6 to 24 carbon atoms. In the above-described general formula (3), $R^{18}$ and $R^{19}$ are the same or different, and represent an alkyl group having 1 to 6 carbon atom(s) or $-(AO)_fH$ ("A" represents an alkylene group having 2 to 4 carbon atoms, and "f" represents an integer number of 1 to 6, and f groups of A may be the same or different, and the sequence thereof is arbitrary). "e" represents an integer number of 1 to 5.

More specifically, this typically includes hexadecyloxy(2-hydroxypropyl) dimethylamine and octadecyloxy(2-hydroxypropyl) dimethylamine.

(ii) ether amine

For example, compounds represented by the following general formula (4) are included.

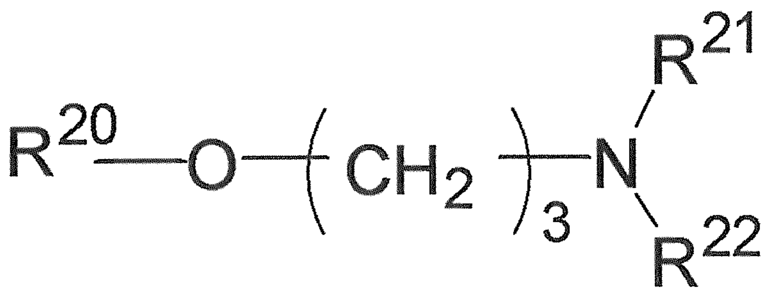

(4)

In the above-described general formula (4), $R^{20}$ represents a linear or branched alkyl group or alkenyl group having 6 to 24 carbon atoms. In the above-described general formula (4), $R^{21}$ and $R^{22}$ are the same or different, and represent an alkyl group having 1 to 6 carbon atom(s) or -(AO)gH ("A" represents an alkylene group having 2 to 4 carbon atoms, and "g" represents an integer number of 1 to 6, and g groups of A may be the same or different, and the sequence thereof is arbitrary).

More specifically, N,N-dimethyl-3-hexadecyloxypropyl amine and N,N-dimethyl-3-octadecyloxypropyl amine are exemplified.

(iii) alkylamide amine

For example, compounds represented by the following general formula (5) are included.

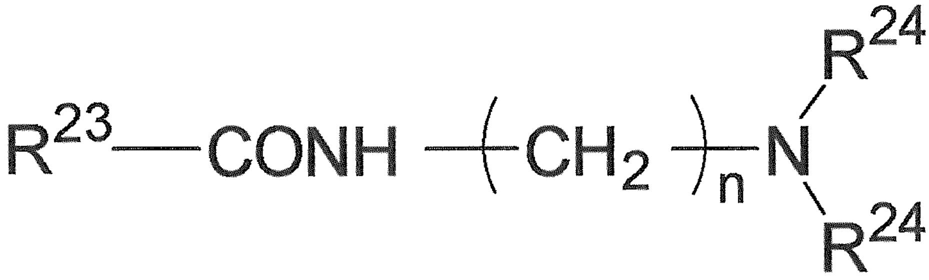

(5)

In the above-described general formula (5), $R^{23}$ represents an aliphatic hydrocarbon group having 11 to 23 carbon atoms. $R^{24}$s are the same or different, and represent an atomic hydrogen or alkyl group having 1 to 4 carbon atoms. "n" represents an integer number of 2 to 4.

More specifically, N-(3-(dimethylamino)propyl) docosanamide and N-(3-(dimethylamino)propyl) stearamide are exemplified.

As a preferable tertiary amine compound of the component (C) selected among the above-described (i) to (iii), either (ii) ether amine or (iii) alkylamide amine is preferable, in view of providing the smoothness in the applying and in the rinsing. Among these, (ii) ether amine is preferable, and N,N-dimethyl-3-hexadecyloxypropyl amine and N,N-dimethyl-3- octadecyloxy propyl amine are more preferable, and N,N-dimethyl-3-octadecyloxy propyl amine is further preferable.

One type of tertiary amine of component (C) may be employed, or two or more types thereof may be jointly employed. In view of providing the smoothness in the rinsing and providing the smoothness after the drying, the content of the component (C) in the vesicle composition is preferably 0.5 to 15 % by mass, more preferably 1 to 10 % by mass, and further preferably 3 to 6 % by mass.

The component (D) employed in the present invention is an organic acid having 1 to 8 carbon atom(s), and more preferably an organic acid having 2 to 6 carbon atoms. More specifically, this includes: monocarboxylic acids such as acetic acid, propionic acid, caprylic acid and the like; dicarboxylic acids such as malonic acid, succinic acid, glutaric acid, adipic acid, maleic acid, fumaric acid and the like; hydroxy carboxylic acids such as glycolic acid, lactic acid, hydroxy acrylic acid, glyceric acid, malic acid, tartaric acid, citric acid and the like; aromatic carboxylic acids such as benzoic acid, salicylic acid, phthalic acid and the like; acidic amino acids such as glutamic acid, aspartic acid and the like. Among these, hydroxy carboxylic acids and acidic amino acids are preferable. More preferable hydroxy carboxylic acid includes, glycolic acid, citric acid, lactic acid, and malic acid, and among these, glycolic acid and lactic acid are preferable. More preferable acidic amino acid includes glutamic acid.

The content of the component (D) in the vesicle composition is preferably equal to or higher than 0.05 % by mass and more preferably equal to or higher than 0.1 % by mass, and on the other hand, is preferably equal to or lower than 4 % by mass and more preferably equal to or lower than 2 % by mass.

In the present invention, water is employed. It is preferable to employ a pure water. The content of water in the vesicle composition is not limited, and may be suitably adjusted for the use according to the applications.

The above-described compounds can be suitably combined to provide a suitable combination of the component (A), the component (B), the component (C) and the component (D). Although it is not intended to limit the specific combination, a preferable combination of the component (A), the component (B), the component (C) and the component (D) may be, for example, a combination of: linear stearic acid for the component (A); isostearic acid (more preferably, ISOSTEARIC ACID EX) for the component (B); (ii) ether amine or (iii) alkylamide amine for the component (C); and glycolic acid or lactic acid for the component (D).

The combination of the component (A), the component (B), the component (C) and the component (D) and water forms the vesicle. In particular, the vesicle composition containing a multilamellar vesicle constituted of several double membranes (so-called onion-vesicle) dispersed in water are easy to be formed. In the mean time, while the vesicle ordinarily means coacervates having a hollow or aqueous-phase internal layer, the multilamellar vesicle formed here also generally includes coacervates having the constitution, in which a part of or the entire of the internal layer is an oil phase. Further, in the present invention, the "vesicle" also includes the multilamellar vesicle.

In view of providing an increased volumetric concentration of the vesicle in the vesicle dispersion, the molar ratio ((A) + (B))/ (D) of the fatty acid (component (A) + component (B)) with the organic acid (component (D)) is preferably equal to or higher than 5/5 and more preferably equal to or higher than 7/3, and preferably equal to or lower than 9/1 and more preferably equal to or lower than 8/2.

Also, in view of achieving effective contribution of the component (A), the component (B), the component (C) and the component (D) for creating the vesicle, the ratio ((A) + (B) + (D)) / (C) of the acid equivalent of (A) + (B) + (D) with the base equivalent of (C) is preferably equal to or higher than 0.25 and more preferably equal to or higher than 0.5 and further preferably equal to or higher than 0.6, and, preferably equal to or lower than 4 and more preferably equal to or lower than 2 and further preferably equal to or lower than 1.8.

In view of enhancing the storage stability and the handling ability of the vesicle dispersion, the summation of the component (A), the component (B), the component (C) and the component (D) in the vesicle composition is preferably 1 to 20 % by mass, and more preferably 1 to 15 % by mass.

In the step (I), the oil phase is necessary to be melted in view of achieving the stable production. Thus, the oil phase is melted at a temperature of equal to or higher than the melting point of the oil phase, and is preferably melted at a temperature of 5 degrees C or more higher than the melting point of the oil phase, and is more preferably melted at a temperature of 10 degrees C or more higher than the melting point of the oil phase.

It is also preferable that the oil phase is the uniformly mixed state. Thus, it is preferable that the oil phase is melted while being mixed in the present process step. The method for mixing is not limited, and for example, it is preferable to mix by stirring.

In the step (II), the temperature in the process of dropping the aqueous phase may be suitably defined by the temperature of the oil phase, the temperature of the aqueous phase added dropwise, and the heating or the cooling by means of the mixing equipment. Here, purified water such as ion-exchange water, distilled water or the like may be employed for the "aqueous phase". Moreover, in view of achieving the effective production of the vesicle, it is preferable to provide the temperature of the oil phase and the temperature of the dropped aqueous phase to be a temperature of equal to or higher than the phase transition temperature of the vesicle.

The volumetric concentration of the vesicle in the vesicle dispersion can be adjusted by suitably adjusting the dropping speed of the aqueous phase to the oil phase and stirring speed when the aqueous phase is added dropwise, and the particle diameter of the vesicle can be adjusted by the stirring speed (shearing velocity) after the dropping of the aqueous phase is started. The aqueous phase is dropped to the oil phase to start the formation of the vesicle, so that the viscosity of the vesicle dispersion is increased and the volumetric concentration of the vesicle is also increased. After the viscosity of the vesicle dispersion is reached to the peak, the dropping of the aqueous phase is further continued, so that the viscosity of the vesicle dispersion is decreased and the volumetric concentration of the vesicle is also decreased. The quantity of the dropped aqueous phase can be suitably adjusted in consideration of the storage stability and the handling ability of the vesicle dispersion.

In the step (II), the aqueous phase is dropped to the oil phase that is the shear mixing state. Thus, the mixing equipment is not limited as long as the equipment can achieve the shear mixing, and when the mixture exhibits higher viscosity in the process of adding the aqueous phase, it is preferable to employ an apparatus which is applicable for mixing the higher viscosity product, such as, for example: AGI HOMO MIXER and TK COMBI MIX, commercially available from PRIMIX Corporation; Vacuum Emulsification Stirring Apparatus, commercially available from MIZUHO Industrial Co., Ltd.; Glasslining Maxblend, commercially available from Sumitomo Heavy Industries, Ltd.; Super Mix Stirring Vessel commercially available from Satake Chemical Equipment Mfg Ltd., and the like.

Optimum values of the dropping speed of the aqueous phase to the oil phase and the stirring speed when the aqueous phase is added dropwise depends upon the formula and the component ratio of the vesicle composition and the size and the shape of the blending vessel, and it is preferable to suitably select these conditions so as to achieve the uniform mixing even in the state that the viscosity is most increased during the dropping of the aqueous phase.

More specifically, the dropping speed of the aqueous phase to the oil phase may be preferably adjusted so that the dropping continues for equal to or longer than 10 minutes, in order to increase the volumetric concentration of the vesicle in the vesicle dispersion. Though it is not intended to limit the dropping speed, it is preferable to carry out the dropping at a rate of, for example, 5 to 20 g/min., when the whole quantity of the dropping aqueous phase is 600 g. Though it is not intended to limit the stirring speed, it is preferable to carry out the stirring at a speed of, for example, 50 to 100 rpm.

The vesicle composition of the present invention may further contain (E) polyhydric alcohol. The component (E) include, more specifically, propylene glycol, dipropylene glycol, 1,3-propanediol, glycerol, 1,3-butylene glycol, isopentyl diol, sorbitol and the like. More preferably, propylene glycol or dipropylene glycol is preferable.

When the component (E) is added, the oil phase containing the component (A), the component (B), the component (C) and the component (D) is melted at a temperature of equal to or higher than the melting point of the oil phase in the above-described step (I), and then the component (E) may be added to the oil phase. Alternatively, the oil phase containing the component (A), the component (B), the component (C), the component (D) and the component (E) may be melted at a temperature of equal to or higher than the melting point of the oil phase until no solid matter is remained to obtain the oil phase. After the step (I), the step (II) for adding the aqueous phase to the obtained oil phase while mixing the solution is conducted to obtain the vesicle dispersion of higher vesicle volumetric concentration and higher storage stability. Besides, when a polyalcohol, which is capable of being dissolved in water, such as, for example, glycerol, dipropylene glycol and the like is employed as the component (E), this may be contained in water.

In addition to the specific combination of the component (A), the component (B), the component (C) and the component (D), more specifically, the combination of: linear stearic acid for the component (A); isostearic acid (more preferably, ISOSTEARIC ACID EX) for the component (B); (ii) ether amine or (iii) alkylamide amine for the component (C); and glycolic acid or lactic acid for the component (D), the component (E) may be added, and the combination with propylene glycol or dipropylene glycol as the component (E) may be more preferable.

The content of the component in the vesicle composition (E) is preferably 0.5 to 60 % by mass in view of the storage stability of the vesicle composition, and more preferably 1 to 50 % by mass, and further preferably 2 to 20 % by mass.

In addition to above, an optional component may be additionally contained in the oil phase without obstructing the production of the vesicle of the present invention. Typical optional component includes, for example, various types of extracts and antioxidants and the like, though it is not limited thereto. The optional component, which is able to be added to the oil phase, is equal to or lower than 1 % by mass of the whole oil phase, in view of achieving the stable production of the vesicle composition.

In addition, an optional component may be additionally contained in the aqueous phase without obstructing the

production of the vesicle of the present invention. Typical component, which can be added thereto, includes, for example, various types of extracts and preservatives and the like, though it is not limited thereto. The amount of the optional component, which is able to be added to the aqueous phase, is equal to or lower than 0.1 % by mass of the whole aqueous phase, in view of the stable production of the vesicle composition.

However, if a higher alcohol (an alcohol having 8 to 24 carbon atoms) is contained in the oil phase or the aqueous phase, layered lamella structure is easily formed in the above-described step (II), instead of forming the vesicle. Thus, higher alcohol is preferably contained at equal to or lower than 2 % by mass in the vesicle composition, and more preferably equal to or lower than 1 % by mass, and further preferably equal to or lower than 0.5 % by mass, and the vesicle composition may preferably contain no or substantially no higher alcohol.

In addition, in view of ensuring the stability of the vesicle composition, a process step for rapidly cooling thereof to a temperature of equal to or lower than the phase transition temperature of the vesicle may be additionally conducted after the dropping of the aqueous phase is ended in the step (II).

It is preferable that the vesicle in the vesicle composition thus produced is in the form of the particulate, and may be in various types of shape such as spherical, oval shape, cylindrical shape, disc shape, indefinite shape and the like. The mean particle diameter of the vesicle is equal to or larger than 5 μm in view of further improving the conforming feel when applying to hair, and is preferably equal to or larger than 7 μm and more preferably equal to or larger than 10 μm, and on the other hand, is equal to or smaller than 500 μm, and is preferably equal to or smaller than 300 μm, and more preferably equal to or smaller than 200 μm, and further preferably equal to or smaller than 50 μm. Here, the mean particle diameter of the vesicle can be measured by employing a device for measuring particle size distribution employed for the above-described measurement of the vesicle volumetric concentration such as, for example, Multisizer™4 commercially available from Beckman Coulter, Inc., CDA-1000 X commercially available from SYSMEX CORPORATION and the like, or a laser diffraction device for measuring particle size distribution such as, for example, SALD2100 commercially available from of SHIMADZU CORPORATION Co., Ltd., LA-920 commercially available from HORIBA, Ltd. and the like, in which an intensity distribution of the scattered light, which is obtained by irradiating the vesicle moving in the flow cell in circulation type device with laser beam, is measured, and the obtained intensity distribution is converted to obtain the volumetric distribution, which is employed to eventually determine the mean particle diameter. Such measurement may be preferably conducted at the room temperature (15 to 30 degrees C).

It is considered that the vesicle obtained by such method easily changes from the vesicle form to the film form when it is applied over the hair, to allow suitably changing its property on the surface of the hair.
The conventional vesicle structure is constituted by containing a specific lipid such as sterols, phospholipid and the like existing therein, as described in, for example, Japanese Patent Domestic Publication No. 2002-516,831. On the contrary, according to the present invention, the vesicle composition can be created even if none of sterols and phospholipid is contained. More specifically, the vesicle structure can be constituted in only the components conventionally employed for the hair cosmetics such as rinse, conditioner and the like.

The hair cosmetic of the present invention contains the above-described vesicle composition.
The content of the vesicle composition in the hair cosmetic may be defined so that the total amount of the component (A) straight chain fatty acid and the component (B) branched fatty acid constituting the vesicle is preferably contained at 0.01 to 5 % by mass and more preferably 0.05 to 2 % by mass in the hair cosmetic, in view of providing a hair cosmetic, which achieves the flexibility, the smoothness, the moist feeling and the like, and more specifically achieving the smoothness and the absence of the slimy feel in the rinsing and also achieving the smoothness and the absence of the pastiness after the drying. Even if the content of the active constituents of such hair cosmetic is reduced as compared with the conventional hair cosmetic, the levels of the flexibility, the smoothness, the moist feeling, and the supple feeling, which are achieved by using the conventional hair cosmetic, can be maintained, or even improved, and on the other hand, smooth and not slimy feeling is achieved in the rinsing, and smooth and not pasty feeling is achieved after the drying.

Such hair cosmetic typically includes, for example, hair conditioners, rinses, hair treatments, shampoos and the like. Hair conditioners, rinses and hair treatments are preferable as more effective hair cosmetics. These hair cosmetics may be either in the type of usage for being washed away or in the type of usage for being not washed away after the hair cosmetic is applied.

The hair cosmetic containing the vesicle composition is obtainable by adding and mixing the vesicle composition of the present invention with a hair cosmetic, which is separately prepared by an ordinary process. This hair cosmetic prepared by the ordinary process is referred to as a base mixture containing, for example, one, two or more cationic surfactant(s) and an aliphatic alcohol, and hair cosmetics containing a silicone, oily ingredient or the like are also included in such hair cosmetic. This can be prepared by any method.

The cationic surfactant employed for the base mixture may typically include quaternary ammonium compounds and tertiary amine compounds, and more preferably, tertiary amine compounds are preferable. A tertiary amine compound is selected from the compounds exemplified above for the component (C). Further, it is preferable to employ the component that is identical to the tertiary amine compound employed for the vesicle composition.

Aliphatic alcohols having 12 to 26 carbon atoms are preferable for the aliphatic alcohol employed for the base

mixture. This allows providing the smoothness for the hair by applying thereof over the hair. It is preferable to employ aliphatic alcohols having a linear or branched alkyl group or alkenyl group, and among these, aliphatic alcohols having a linear or branched alkyl group or alkenyl group having 16 to 22 carbon atoms are preferable. Further, aliphatic alcohols having a linear or branched alkyl group or alkenyl group having 16 to 18 carbon atoms are more preferable. More specifically, cetyl alcohol or stearyl alcohol is preferable.

Although the formula and the preparation process of the hair cosmetic are not limited, the hair cosmetic is obtainable by, for example, adding the oil phase containing the cationic surfactant and the aliphatic alcohol to the heated and stirred aqueous phase to cause the emulsification. The oil phase may contain an emulsified silicone. Typical method for blending the vesicle composition of the present invention into the hair cosmetic may be a method including a process step for mixing the above-described vesicle composition with a base mixture containing at least one type of cationic surfactant and the aliphatic alcohol, and in such case, it is preferable to blend at a temperature of equal to or lower than the phase transition temperature of the vesicle, in view of the stability of the vesicle. This allows obtaining the hair cosmetic having the maintained structure of the vesicle composition.

## EXAMPLES

### Example 1A

Vesicle compositions (premixes) were obtained by the following procedure.
4.2 g of stearic acid (LUNAC S-90V: commercially available from Kao Corporation); 2.8 g of isostearic acid (ISOSTEARIC ACID EX: commercially available from KOKYU ALCOHOL KOGYO CO., LTD.); 0.60 g of lactic acid (MUSASHINO LACTIC ACID 90: commercially available from Musashino Chemical Laboratory Co., Ltd.); and 12.0 g of FARMIN DME-80 (N,N-dimethyl-3-octadecyloxypropyl amine: commercially available from Kao Corporation) were supplied into a 300 ml beaker, and the mixture was heated to 80 degrees C while being stirred with a propeller so that the raw materials were completely dissolved. 180.4 g of ion-exchange water heated to 80 degrees C as an aqueous phase was dropped to this oil phase at constant dropping speed for ten minutes to achieve the emulsification at 80 degrees. Thereafter, the mixture was cooled to a temperature of equal to or less than 35 degrees C. Thus obtained vesicle composition was utilized as a premix. The procedure for achieving the emulsification by dropping the aqueous phase while stirring the oil phase in this way is generally referred to as a phase inversion emulsification. The conditions for the production of the premix and the relative quantities (g) of the respective components converted into the premix 10.00 g were shown in Table 1. In Table 1, percent (%) indicates "% by mass".

### Examples 2A to 11A, Comparative Examples 1B and 2B

The vesicle compositions were obtained according to the formulae shown in Table 1, similarly as in Example 1A. Meanwhile, the following products were employed for the component (A), the component (B), and the component (E).
Palmitic acid: LUNAC P-95, commercially available from Kao Corporation;
Myristic acid: LUNAC MY-98, commercially available from Kao Corporation;
16-methyloctadecane acid: synthesized by the preparation process described in Japanese Laid-Open Patent Application Publication No. 2008-255,050, Example 1 ; and
Dipropylene glycol: DPG-RF, commercially available from ADEKA CORPORATION.

### Examples 1 to 13, Comparative Examples 1 to 2

Here, the premixes, which are the prepared vesicle compositions, were employed to prepare the hair conditioners by the method described below, and the respective evaluations were conducted. The formulae and the evaluation results of Examples 1 to 13 and Comparative Examples 1 to 2 are shown in Table 2. In addition to above, the hair conditioners being set in Examples 1 to 13 and Comparative Examples 1 to 2 were the hair cosmetics employed in the form of being washed away.

### Hair Conditioner

167.8 g of ion-exchange water and 0.69 g of 90 % by mass lactic acid (MUSASHINO LACTIC ACID 90: commercially available from Musashino Chemical Laboratory, Ltd.) were supplied to a 300 ml beaker to prepare an aqueous phase, and the aqueous phase was heated up to 55 degrees C while stirring with a propeller. Then, an oil phase composed of: 2.45 g of N,N-dimethyl-3-octadecyloxy propyl amine (FARMIN DME-80: commercially available from Kao Corporation); 5.58 g of stearyl alcohol (KALCOL 8098: commercially available from Kao Corporation); and 0.93 g of dipropylene glycol (DPG-RF: commercially available from ADEKA CORPORATION) was uniformly melted at

80 degrees C, and then the oil phase was added to the aqueous phase and was stirred at 300 rpm for ten minutes to carry out the emulsification. The emulsion was radiationally-cooled to a temperature of equal to or less than 35 degrees C while being stirred at 300 rpm to prepare a base mixture, and then 7.14 g of the above-mentioned vesicle composition (premix) was added to provide a hair conditioner.
Meanwhile, in Examples 4 and 6, hair rinses were prepared by the similar procedure as statement above, except that 2.29 g of N-(3-(dimethylamino)propyl) stearamide (Amidoamine MPS: commercially available from Nikko Chemicals Co., Ltd.) was in place of adding 2.45 g of N,N-dimethyl-3-octadecyloxy propyl amine.

Measurements of Congealing Points for Mixtures of Component (A) and Component (B)

Thermally melted mixture of the component (A) and the component (B) was precisely weighed and was enclosed in a cell for measurements to prepare a sample, and the congealing point was measured by employing a differential scanning calorimetry (EXSTAR6000: commercially available from Seiko Instruments Inc.). The measurement conditions were that the temperature was elevated from 5 degrees C to 90 degrees C at the rate of temperature elevation of 1 degree C/min., and then the sample was cooled to 5 degrees C at the rate of cooling of 1 degree C/min. The congealing point was determined from the position where the heat generation peak in cooling appeared.

Observations of Vesicle Structure in Premix

The premixes were observed at a room temperature under the polarized light condition (crossed nicol) by employing an optical microscope (ECLIPSE E800: commercially available from NIKON CORPORATION). In addition, the mean particle diameters of the vesicles in the premixes were measured at 25 degrees C by employing Multisizer™4 commercially available from Beckman Coulter, Inc. In addition to above, the median diameter (D50) of volumetric reference was adopted for the mean particle diameter.

Feel Evaluation

Hair of a Japanese woman, which had been subjected to treatment such as one straight permanent and twice bleaching, was employed as damaged hair, and each of 20 g of respective hair bundles (15 to 20 cm long, average diameter of 80 $\mu$m) was cleaned by using 2 g of a standard shampoo having the following formulation, and then each of 2 g of hair conditioners shown in Table 2 was applied over the hair bundle and after the conditioner was sufficiently spread over the entire hair, the hair was rinsed out with running water at about 40 degrees C for about 30 seconds, and thereafter was dried with a towel and eventually sufficiently dried with a dryer.

* Formula of Standard Shampoo (pH 7.0)

(hereinafter, the percentage (%) represents % by mass.) 25 % sodium polyoxyethylene (2.5) lauryl ether sulfate: 62.0%; lauric diethanolamide: 2.3%;
edetate disodium: 0.15%;
sodium benzoate: 0.5%;
sodium chloride: 0.8%;
75 % phosphoric acid: proper amount;
flavor, methylparaben: proper amount; and
purified water: residual quantity.

Evaluations for "Smoothness in Rinsing," "Absence of slimy feel in Rinsing," "Smoothness after drying," and "Absence of Pastiness after Drying" of the hair were carried out. The evaluations were made with six people by utilizing four-point scale, and summation of the points of the evaluations was represented.

(Criteria for Evaluation)

"Smoothness in Rinsing"
4: very smooth;
3: moderately smooth;
2: not very smooth;
1: not smooth.

"Absence of Slimy Feel in Rinsing"
4: absolutely no slimy feel in hair;
3: not very slimy feel in hair;

2: some slimy feel in hair;
1: slimy feel in hair.

"Smoothness after Drying"

4: very smooth;
3: moderately smooth;
2: not very smooth;
1: not smooth.

"Absence of Pastiness after Drying"

4: not pasty;
3: hardly pasty;
2: moderately pasty;
1: pasty.

Table-1 VESICLE COMPOSITION

| | | EXAMPLE | | | | | | | | | | | COMPARATIVE EXAMPLE | |
|---|---|---|---|---|---|---|---|---|---|---|---|---|---|---|
| | | 1A | 2A | 3A | 4A | 5A | 6A | 7A | 8A | 9A | 10A | 11A | 1B | 2B |
| COMPONENT(A) | STEARIC ACID | 2.10 | 2.45 | 2.10 | 1.75 | 1.40 | | | 1.75 | 2.10 | 2.10 | 2.10 | - | 0.70 |
| | PALMITIC ACID | | | | | | 2.10 | | | | | | | |
| | MYRISTIC ACID | | | | | | | 2.10 | | | | | | |
| COMPONENT(B) | ISOSTEARIC ACID (ISOSTEARIC ACID EX) | 1.40 | 1.05 | 1.40 | 1.75 | 2.10 | 1.40 | 1.40 | | 1.40 | 1.40 | 1.40 | 3.50 | 2.80 |
| | 16-METHYLOCTADECANE ACID | | | | | | | | 1.75 | | | | | |
| COMPONENT(C) | N,N-DIMETHYL-3-OCTADECYLOXYPROPYL AMINE | 6.00 | 6.00 | 6.00 | 6.00 | 6.00 | 6.40 | 6.80 | 6.00 | 6.00 | 6.00 | 6.00 | 5.94 | 6.00 |
| COMPONENT(D) | LACTIC ACID 90 % | 0.30 | 0.30 | 0.30 | 0.30 | 0.30 | 0.32 | 0.35 | 0.32 | 0.70 | 0.20 | | 0.30 | 0.30 |
| | GLYCOLIC ACID 70 % | | | | | | | | | | | 0.40 | | |
| COMPONENT(E) | DIPROPYLENE GLYCOL | - | 10.5 | 10.5 | 10.5 | 10.5 | 10.5 | 10.5 | 10.5 | 10.5 | 10.5 | 10.5 | 10.5 | 10.5 |
| | PURIFIED WATER | BALANCE | BALANCE | BALANCE | BALANCE | BALANCE | BALANCE | BALANCE | BALANCE | BALANCE | BALANCE | BALANCE | BALANCE | BALANCE |

(continued)

| | | EXAMPLE | | | | | | | | | | | COMPARATIVE EXAMPLE | |
|---|---|---|---|---|---|---|---|---|---|---|---|---|---|---|
| | | 1A | 2A | 3A | 4A | 5A | 6A | 7A | 8A | 9A | 10A | 11A | 1B | 2B |
| EVALUATIONS | (A)/(B) | 1.50 | 2.33 | 1.50 | 1.00 | 0.67 | 1.50 | 1.50 | 1.00 | 1.50 | 1.50 | 1.50 | 0.00 | 0.25 |
| | (A)+(B)+(C)+(D) | 9.8 | 9.8 | 9.8 | 9.8 | 9.8 | 10.2 | 10.7 | 9.8 | 10.2 | 9.7 | 9.5 | 9.7 | 9.8 |
| | ((A)+(B)+(D)) /(C) | 1.0 | 1.0 | 1.0 | 1.0 | 1.0 | 1.0 | 1.0 | 1.0 | 1.2 | 0.9 | 1.0 | 1.0 | 1.0 |
| | ((A)+(B))/(D) | 4.1 | | | | | | | | 1.8 | 6.2 | 3.3 | | |
| | CONGEALING POINT OF MIXTURE (A)(B) (°C) | 49 | 55 | 49 | 50 | 47 | 53 | 42 | 55 | 49 | 49 | 49 | <0 | 38 |
| | EXISTENCE OR NON-EXISTENCE OF VESICLE(ROOM TEMPERATURE) | EXISTENCE | EXISTENCE | EXISTENCE | EXISTENCE | EXISTENCE | EXISTENCE | EXISTENCE | EXISTENCE | EXISTENCE | EXISTENCE | EXISTENCE | NON-EXISTENCE | NON-EXISTENCE |
| | MEAN PARTICLE DIAMETER OF VESICLE ($\mu$m) | 13.5 | 15.0 | 11.3 | 10.6 | 11.5 | 11.3 | 10.2 | 14.2 | 10.7 | 7.3 | 11.0 | - | - |

(A)/(B) represents mass ratio.
[(A)+(B)+(D)]/(C) represents ratio of summation of acid equivalent with base equivalent.
[(A)+(B)]/(D) represents molar ratio.

Table-2 HAIR CONDITIONER

| | | | EXAMPLE | | | | | | | | | | | | | COMPARATIVE EXAMPLE | |
|---|---|---|---|---|---|---|---|---|---|---|---|---|---|---|---|---|---|
| | | | 1 | 2 | 3 | 4 | 5 | 6 | 7 | 8 | 9 | 10 | 11 | 12 | 13 | 1 | 2 |
| FORMULATIONS | N,N-DIMETHYL-3-OCTADECYLOXYPROPYL AMINE | | 2.45 | 2.45 | 2.45 | | 2.45 | | 2.45 | 2.45 | 2.45 | 2.45 | 2.45 | 2.45 | 2.45 | 2.45 | 2.45 |
| | N-(3-(DIMETHYLAMINO)PROPYL) STEARAMIDE | | | | | 2.29 | | 2.29 | | | | | | | | | |
| | STEARYL ALCOHOL | | 5.58 | 5.58 | 5.58 | 5.58 | 5.58 | 5.58 | 5.58 | 5.58 | 5.58 | 5.58 | 5.58 | 5.58 | 5.58 | 5.58 | 5.58 |
| | DIPROPYLENE GLYCOL | | 0.93 | 0.93 | 0.93 | 0.93 | 0.93 | 0.93 | 0.93 | 0.93 | 0.93 | 0.93 | 0.93 | 0.93 | 0.93 | 0.93 | 0.93 |
| | LACTIC ACID 90 % | | 0.69 | 0.69 | 0.69 | 0.69 | 0.69 | 0.69 | 0.69 | 0.69 | 0.69 | 0.69 | 0.69 | 0.69 | 0.69 | 0.69 | 0.69 |
| | VESICLE COMPOSITION | 1A | 7.14 | | | | | | | | | | | | | | |
| | | 2A | | 7.14 | | | | | | | | | | | | | |
| | | 3A | | | 7.14 | 7.14 | | | | | | | | | | | |
| | | 4A | | | | | 7.14 | 7.14 | | | | | | | | | |
| | | 5A | | | | | | | 7.14 | | | | | | | | |
| | | 6A | | | | | | | | 7.14 | | | | | | | |
| | | 7A | | | | | | | | | 7.14 | | | | | | |
| | | 8A | | | | | | | | | | 7.14 | | | | | |
| | | 9A | | | | | | | | | | | 7.14 | | | | |
| | | 10A | | | | | | | | | | | | 7.14 | | | |
| | | 11A | | | | | | | | | | | | | 7.14 | | |
| | | 1B | | | | | | | | | | | | | | 7.14 | |
| | | 2B | | | | | | | | | | | | | | | 7.14 |
| | PURIFIED WATER | | BALANCE | BALANCE | BALANCE | BALANCE | BALANCE | BALANCE | BALANCE | BALANCE | BALANCE | BALANCE | BALANCE | BALANCE | BALANCE | BALANCE | BALANCE |
| EVALUATIONS | SMOOTHNESS IN RINSING | | 22 | 23 | 24 | 20 | 23 | 19 | 22 | 22 | 22 | 20 | 20 | 16 | 22 | 7 | 8 |
| | ABSENCE OF SLIMY FEEL IN RINSING | | 21 | 22 | 23 | 24 | 22 | 24 | 22 | 23 | 22 | 19 | 23 | 18 | 20 | 12 | 12 |
| | SMOOTHNESS IN DRYING | | 22 | 23 | 24 | 23 | 22 | 23 | 22 | 22 | 21 | 24 | 20 | 18 | 23 | 6 | 7 |
| | ABSENCE OF PASTINESS IN DRYING | | 21 | 22 | 24 | 24 | 22 | 24 | 22 | 22 | 23 | 24 | 22 | 19 | 20 | 4 | 6 |

## Claims

1. A process for preparing a vesicle composition having a continuous phase constituted of an aqueous phase, comprising:

   melting an oil phase at a temperature of equal to or higher than a melting point of the oil phase, the oil phase comprising following components (A) to (D):

   component (A) straight chain fatty acid having 14 to 24 carbon atoms;
   component (B) branched fatty acid having 14 to 24 carbon atoms and represented by a general formula (1);

   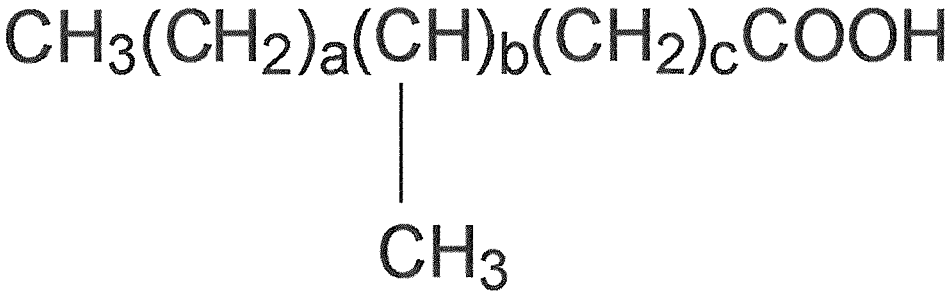

   (1)

   wherein, in the formula, summation of a, b and c is: a + b + c = 11 to 21, and b is 1;
   component (C) tertiary amine compound; and
   component (D) organic acid having 1 to 8 carbon atom(s),
   mass ratio of the component (A) and the component (B) being: (A)/(B) = 70/30 to 40/60, and

   adding the melted oil phase to the aqueous phase while mixing thereof.

2. The process for preparing the vesicle composition according to claim 1, wherein a congealing point of a mixture of the component (A) and the component (B) is equal to or higher than 40 degrees C and equal to or lower than 55 degrees C.

3. The process for preparing the vesicle composition according to Claim 1 or 2, wherein the oil phase or the aqueous phase further comprises (E) polyalcohol.

4. The process for preparing the vesicle composition according to any one of Claims 1 to 3, wherein summation of the components (A), (B), (C) and (D) in the vesicle composition is 1 to 20 % by mass.

5. The process for preparing the vesicle composition according to any one of Claims 1 to 4, wherein, concerning the components (A), (B) and (D), molar ratio of molar summation of the components (A) and (B): (A) + (B), with molar number of the component (D), is presented as:

$$((A) + (B))/(D) = 5/5 \text{ to } 9/1.$$

6. The process for preparing the vesicle composition according to any one of Claims 1 to 5, wherein, concerning the components (A), (B), (C) and (D), ratio of summation of acid equivalents of the components (A), (B) and (D): (A) + (B) + (D) with base equivalent of the component (C) is presented as:

$$((A) + (B) + (D))/(C) = 0.25 \text{ to } 4.$$

7. The process for preparing the vesicle composition according to any one of Claims 1 to 6, wherein, mass ratio of the component (A) with the component (B): (A)/(B) is presented as: (A)/(B) = 60/40 to 50/50.

8. The process for preparing the vesicle composition according to any one of Claims 1 to 7, wherein, the component

(B) is a branched fatty acid having 16 to 22 carbon atoms.

**9.** The process for preparing the vesicle composition according to any one of Claims 1 to 8, wherein, carbon number of fatty acid of the component (A) is equivalent to that of the component (B).

**10.** The process for preparing the vesicle composition according to any one of Claims 1 to 9, wherein, the component (A) is stearic acid,
the component (B) is isostearic acid,
the component (C) is ether amine or alkylamide amine, and
the component (D) is glycolic acid or lactic acid.

**11.** A process for preparing a hair cosmetic, comprising:

melting an oil phase at a temperature of equal to or higher than a melting point of the oil phase, the oil phase comprising following components (A) to (D):

component (A) straight chain fatty acid having 14 to 24 carbon atoms;
component (B) branched fatty acid having 14 to 24 carbon atoms and represented by a general formula (1);

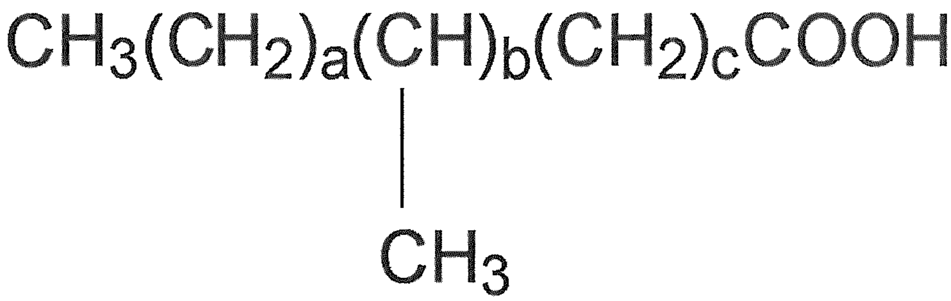

**(1)**

branched saturated fatty acid, wherein, in the formula, summation of a, b and c is: a + b + c = 11 to 21, and b is 1;
component (C) tertiary amine compound; and
component (D) organic acid having 1 to 8 carbon atom(s),
mass ratio of the component (A) and the component (B) being: (A)/(B) = 70/30 to 40/60, and

adding the melted oil phase to the aqueous phase while mixing thereof to produce a vesicle composition having the aqueous phase as a continuous phase; and
mixing the vesicle composition in a base mixture comprising at least a cationic surfactant and an aliphatic alcohol.

**12.** A vesicle composition having a continuous phase constituted of an aqueous phase, comprising components (A), (B), (C) and (D) and water, wherein, mass ratio of the component (A) and the component (B) is: (A)/(B) = 70/30 to 40/60, and the vesicle composition comprises a vesicle, a mean particle diameter of the vesicle being 5 to 500 μm:

(A) straight chain fatty acid having 14 to 24 carbon atoms;
(B) branched fatty acid having 14 to 24 carbon atoms and represented by a general formula (1);

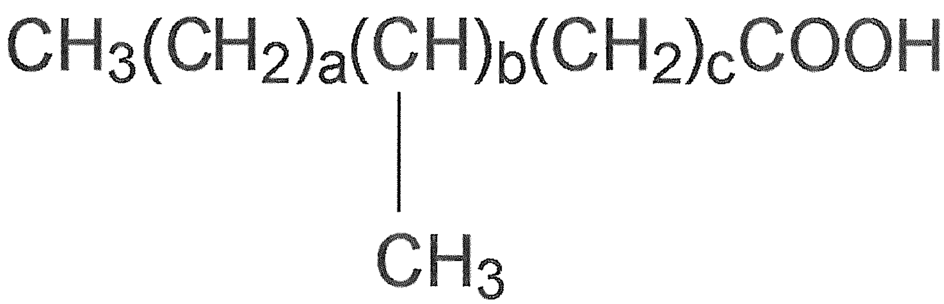

**(1)**

wherein, in the formula, summation of a, b and c is: $a + b + c = 11$ to 21, and b is 1;
(C) tertiary amine compound; and
(D) organic acid having 1 to 8 carbon atom(s).

**13.** A hair cosmetic in a form of a treatment, a conditioner or a rinse, which is obtainable by mixing the vesicle composition according to Claim 12 to a base mixture comprising one type or multiple types of cationic surfactant(s) and an aliphatic alcohol.

**INTERNATIONAL SEARCH REPORT**

International application No.
PCT/JP2012/000283

A. CLASSIFICATION OF SUBJECT MATTER
*A61K8/36*(2006.01)i, *A61K8/14*(2006.01)i, *A61K8/34*(2006.01)i, *A61K8/365*(2006.01)i, *A61K8/41*(2006.01)i, *A61Q5/12*(2006.01)i

According to International Patent Classification (IPC) or to both national classification and IPC

B. FIELDS SEARCHED

Minimum documentation searched (classification system followed by classification symbols)
A61K8/36, A61K8/14, A61K8/34, A61K8/365, A61K8/41, A61Q5/12

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2005-187342 A (Lion Corp.),<br>14 July 2005 (14.07.2005),<br>claims 1, 3; paragraphs [0020], [0021], [0051];<br>example 23<br>(Family: none) | 1-9,11-13<br>10 |
| A | WO 2010/052093 A1 (Unilever N.V.),<br>14 May 2010 (14.05.2010),<br>claims 1, 3 to 5; page 4, lines 1 to 14;<br>examples 1, 2<br>& EP 2344255 A1 | 1-13 |
| A | JP 2010-538038 A (BASF SE),<br>09 December 2010 (09.12.2010),<br>table 6<br>& EP 2197998 A1 & WO 2009/030614 A1<br>& CN 101796175 A & KR 10-2010-0083134 A | 1-13 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
17 April, 2012 (17.04.12)

Date of mailing of the international search report
24 April, 2012 (24.04.12)

Name and mailing address of the ISA/
Japanese Patent Office

Facsimile No.

Authorized officer

Telephone No.

Form PCT/ISA/210 (second sheet) (July 2009)

**INTERNATIONAL SEARCH REPORT**

International application No.
PCT/JP2012/000283

C (Continuation). DOCUMENTS CONSIDERED TO BE RELEVANT

| Category* | Citation of document, with indication, where appropriate, of the relevant passages | Relevant to claim No. |
|---|---|---|
| A | JP 2004-508313 A (The Procter & Gamble Co.), 18 March 2004 (18.03.2004), claim 1; paragraph [0008] & US 2003/0215415 A1 & EP 1328240 A1 & WO 2002/019976 A1 & CN 1454074 A | 1-13 |
| P,A | JP 2011-46634 A (Toyobo Co., Ltd.), 10 March 2011 (10.03.2011), claims 1, 6; table 1 (Family: none) | 1-13 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2008297218 A
- JP 2002114648 A
- JP 2002516831 A
- JP 2008094980 A
- JP 2008255050 A


### Non-patent literature cited in the description


- Optimization of Nano-emulsion Preparation by Low-Energy Methods in an Ionic Surfactant System. *Langmuir,* 2006, vol. 22, 8326-8332
- Cosmetics Raw Material Standard. YAKUJI NIPPO LIMITED, 1984, 87